# EUROPÄISCHE PATENTSCHRIFT

(11) **EP 0 969 807 B1**
(45) Veröffentlichungstag und Bekanntmachung des Hinweises auf die Patenterteilung: **19.01.2005**
(21) Anmeldenummer: 98916988.3
(22) Anmeldetag: 18.03.1998
(51) Int. Cl.: A61K 7/48, A61K 7/06, A61K 7/00, A61K 9/16

(54) **CHITOSANMIKROSPHÄREN**
CHITOSANE MICROSPHERES
MICROSPHERES DE CHITOSANE

(30) Priorität: 27.03.1997 DE 19712978
(43) Veröffentlichungstag der Anmeldung: 12.01.2000
(73) Patentinhaber: Cognis Deutschland GmbH & Co. KG, 40589 Düsseldorf (DE)
(72) Erfinder: WACHTER, Rolf, D-40595 Düsseldorf (DE); HORLACHER, Peter, D-88477 Schwendi (DE)
(86) Internationale Anmeldenummer: PCT/EP1998/001557
(87) Internationale Veröffentlichungsnummer: WO 1998/043609

(56) Entgegenhaltungen:
- WO-A-93/17784
- WO-A-96/00056
- WO-A-96/05810
- DE-A- 4 442 987
- DE-A- 19 604 180

## Beschreibung

### Gebiet der Erfindung

Die Erfindung betrifft Chitosanmikrosphären mit einem Gehalt an lipophilen Stoffen, ein Verfahren zu deren Herstellung sowie deren Verwendung als Wirkstoffdepots zur Einarbeitung in tensidische Formulierungen.

### Stand der Technik

Mikrosphären, die in ihrem Inneren einen oder mehrere Wirkstoffe enthalten und diese zeitverzögert an die äußere Umgebung - in der Regel den menschlichen Körper - abgeben, sind aus dem Stand der Technik bekannt. Üblicherweise ist es zur Herstellung dieser Mikrosphären erforderlich, daß ein Polymer in einem Lösungsmittel aufgelöst oder zumindest suspendiert wird. Damit besteht die Gefahr, daß im Laufe der Herstellung der Produkte eine wenn auch geringe Menge des Lösungsmittels anhaftet. Es ist daher sofort klar, daß an die Auswahl der in Frage kommenden Lösungsmittel, die im folgenden als Trägerphasen bzw. Trägeröle bezeichnet werden, strenge Maßstäbe hinsichtlich ihrer toxikologischen Verträglichkeit gestellt werden müssen. Ein weiteres Problem besteht darin, daß die Herstellung dieser Mikrosphären in aller Regel auch die Extrusion und Feinzerkleinerung der Partikel umschließt, wobei es leicht dazu kommen kann, daß Partikel mit unregelmäßiger Oberfläche entstehen, die für die kontrollierte zeitverzögerte Freisetzung von Wirkstoffen ausgesprochen unvorteilhaft sind. Neben der toxikologischen Unbedenklichkeit müssen die in Betracht kommenden Trägerphasen somit zudem die Herstellung von Mikrosphären mit regelmäßiger Oberflächenbeschaffenheit sicherstellen, so daß sich der freizusetzende Wirkstoff ausschließlich im Inneren der Sphären und nicht an deren Oberfläche befindet.

Aus dem Stand der Technik sind Verfahren zur Herstellung von Partikeln ohne Verwendung eines Lösungsmittels und ohne Einsatz von Extrusions- oder Feinzerkleinerungsmethoden bekannt. So wird z.B. in der **WO 92/21326** die Umwandlung der aus einem Arzneimittelprodukt und biokompatiblen Polymeren bestehenden Mischung in einer flüssigen Zwischenphase durch Erwärmen beschrieben.

Danach wird die besagte Flüssigphase durch Kühlung in eine Feststoffphase umgewandelt und die Grundmasse aus der Feststoffphase ausgewaschen. Letztere liegt auf diese Weise in einer Form vor, die Spuren der kristallinen Struktur der temporären Grundmasse aufweist. Folglich haben die Partikel eine unregelmäßige äußere Oberfläche, sind ganz offensichtlich nicht kugelförmig und weisen somit keine Eigenschaften auf, wie sie für eine kontrollierte Freisetzung des Wirkstoffes erforderlich wären.

In einem Aufsatz von E.Mathiowitz und R.Langer in **J.Controll.Rel. 5, 13 (1987)** wird ein Gemisch aus einem Arzneimittelprodukt und einem geschmolzenen Polymer sowie die Suspension dieses Gemisches in einem Lösemittel beschrieben, in dem das ausgewählte Polymer und das spezielle Arzneimittelprodukt nicht miteinander mischbar sind. Nach Stabilisierung der so gewonnenen Emulsion wird die Mischung bis zum Erstarren abgekühlt. Nach diesem Verfahren handelt es sich bei dem Polymer um einen Stoff mit einem niedrigen Schmelzpunkt bzw. um eine Stoffmischung, die ebenfalls niedrig schmelzend ist, so daß das Verfahren bei niedrigen Temperaturen durchgeführt werden kann. Auf diese Weise lassen sich jedoch keine Mikrosphären herstellen, die ausschließlich hochschmelzende Polymere und Wirkstoffe enthalten, zumal die Durchführung des Verfahrens bei höheren Temperaturen zu einem unerwünschten Verkleben der Inhaltsstoffe führen würde. Schließlich weisen die nach diesem Verfahren erhaltenen Sphären nicht nur eine extrem kömige und daher aus den bereits genannten Gründen unvorteilhafte Oberfläche auf, der niedrige Schmelzpunkt der Polymere kann zudem auch die Lagerstabilität der Mikrosphären nachteilig beeinflussen.

Aus der britischen Offenlegungsschrift **GB-A 2246514** ist ein Verfahren bekannt, bei dem man Mikrosphären in einem Gel herstellt und dabei auf die bekannten Methoden der Extrusion und Feinzerkleinerung zurückgreift. Das Verfahren hat aber in Summe den Nachteil, daß ein großer Anteil des wertvollen Wirkstoffs verloren geht und die Herstellung von Mikrosphären mit einer Beladungsstärke von mehr als 15 Gew.-% nicht möglich ist. Aus den beiden französischen Patentschriften **FR-B1 2665360** und **FR-B1 2705323** (SCRAS) sind Verfahren zur Herstellung von Mikrosphären bekannt, bei dem man biokompatible Polymere in einer Trägerphase aus Siliconöl dispergiert und dann den Wirkstoff einbringt. Es werden in der Tat regelmäßige, sphärische Partikel erhalten, die den Wirkstoff in ihrem Innem enthalten, im Hinblick auf die problematischen toxikologischen Eigenschaften von Siliconölen kommt die Verwendung dieser Trägerphasen jedoch nicht länger in Betracht. Aus der internationalen Patentanmeldung **WO 92/06672** (Revlon) ist schließlich die Verkapselung von Antitranspirantien bekannt, bei der man Polymere wie beispielsweise Chitosan einsetzen kann.

Die WO-A-9 605 810 offenbart die Herstellung von Chitosanmikrosphären, wobei ein Chitosanderivat mit einem Öl vermischt wird und durch Zugabe einer NaOH-Lösung die Chitosanmikrosphären ausgefällt werden.

Demzufolge hat die komplexe Aufgabe der Erfindung darin bestanden, Mikrosphären zur zeitverzögerten Freigabe von Wirkstoffen, vorzugsweise kosmetischen Ölen, zur Verfügung zu stellen, die sich auf einfachstem Wege herstellen lassen, eine regelmäßige Oberfläche besitzen und zudem noch über eine hohe Biokompatibilität verfügen.

### Beschreibung der Erfindung

Gegenstand der Erfindung sind Chitosanmikrosphären, die man erhält, indem man Chitosane und/oder Chitosanderivate mit Ölkörpem vermischt und die Mischungen anschließend in alkalisch eingestellte Tensidlösungen einbringt, so daß die resultierenden Chitosanmikrosphären einen Durchmesser von 1 mm bis 5 mm aufweisen.

Überraschenderweise wurde gefunden, daß beim Einbringen von Mischungen von Chitosanen bzw. Chitosanderivaten mit Ölkörpem in wäßrige, alkalische Tensidlösungen eine Fällung stattfindet und Mikrosphären entstehen, bei denen die Chitosane die lipophilen Komponenten verkapseln. Abgesehen davon, daß die Mikrosphären feine Perlen mit einer sehr regelmäßigen Oberfläche darstellen und somit sehr ästhetisch aussehen, können auf diese Weise Zubereitungen zur Verfügung gestellt werden, die Ölkörper, gegebenenfalls auch lipophile Wirkstoffe, zeitverzögert freigeben und sich problemlos in wäßrigtensidische Formulierungen einarbeiten lassen. Ein weiterer Vorteil besteht in der hohen Biokompatibilität der Chitosane als Träger.

### Chitosane

Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

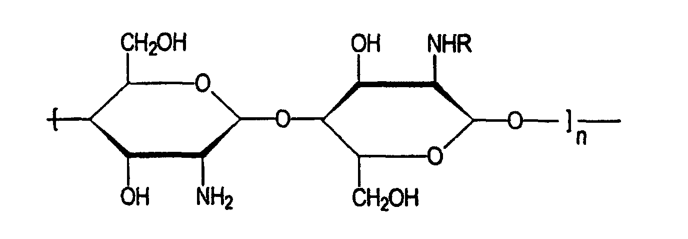

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt (vgl. **Ullmann's Encyclopedia of Industrial Chemistry, 5th Ed., Vol. A6, Weinheim, Verlag Chemie, 1986, S. 231.332).** Übersichten zu diesem Thema sind auch beispielsweise von B.Gesslein et al. in **HAPPI 27, 57 (1990),** O.Skaugrud in **Drug Cosm.lnd. 148, 24 (1991)** und E.Onsoyen et al. in **Seifen-Öle-Fette-Wachse 117, 633 (1991)** erschienen. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren sind beispielsweise aus **Makromol. Chem. 177, 3589 (1976)** oder der französischen Patentanmeldung **FR-A 2701266** bekannt. Vorzugsweise werden solche Typen eingesetzt, wie sie in den deutschen Patentanmeldungen **DE-A1 4442987** und **DE-A1 19537001** (Henkel) offenbart werden, und die ein durchschnittliches Molekulargewicht von 800.000 bis 1.200.000 Dalton, eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% aufweisen. Neben den Chitosanen als typischen kationischen Biopolymeren kommen im Sinne der Erfindung auch anionisch bzw. nichtionisch derivatisierte Chitosane, wie z.B. Carboxylierungs-, Succinylierungs- oder Alkoxylierungsprodukte in Frage, wie sie beispielsweise in der deutschen Patentschrift **DE-C2 3713099** (L'Oréal) sowie der deutschen Patentanmeldung **DE-A1 19604180** (Henkel) beschrieben werden.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN), Dialkylether, Siliconöle und/oder aliphatische bzw. naphthenische Kohlenwasserstoffe in Betracht. Die Mikrosphären können die Chitosane und die Ölkörper im Gewichtsverhältnis 1:10 bis 10 :1, vorzugsweise 1 : 5 bis 1 : 8 enthalten.

### Hilfs- und Zusatzstoffe

Die erfindungsgemäßen Chitosankapseln, können ferner als weitere Hilfs- und Zusatzstoffe Emulgatoren, Überfettungsmittel, Stabilisatoren, Wachse, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Lichtschutzfilter, Insektenrepellentien, Selbstbräuner, Farb- und Duftstoffe enthalten.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Trialkylphosphate sowie Mono-, Di- und/oder Tri-PEG-alkylphosphate;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglucose und Polyolen, vorzugsweise Glycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht.

C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside werden insbesondere durch Umsetzung von Glucose oder Oligosacchariden mit primären Alkoholen mit 8 bis 18 C-Atomen hergestellt. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazofine mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquatemierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Afkyloligoglucäside sowie Elektrolyte wie Kochsalz und Ammoniumchlorid.

Geeignete **kationische Polymere** sind beispielsweise kationische Cellulosederivate, wie z.B. ein quatemierte Hydroxyethylcellulose, die unter der Bezeichnung Polymer JR 400@ von Amerchol erhältlich ist, kationische Stärke, Copolymere von Diallyiammoniumsalzen und Acrylamiden, quatemierte VinylpyrrolidonNinyl-imidazol-Polymere wie z.B. Luviquat® (BASF), Kondensationsprodukte von Polyglycolen und Aminen, quatemierte Kollagenpolypeptide wie beispielsweise Lauryldimonium hydroxypropyl hydrolyzed collagen (Lamequat®L/Grünau), quatemierte Weizenpolypeptide, Polyethylenimin, kationische Siliconpolymere wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide wie z.B. beschrieben in der **FR-A 2252840** sowie deren vemetzte wasserlöslichen Polymere, kationische Chitinderivate wie beispielsweise quatemiertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese, quatemierte Ammoniumsalz-Polymere wie z.B. Mirapol® A-15, Mirapol® AD-1, Mirapol® AZ-1 der Miranol.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Camaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als Pertgtanzwachse können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als Stabilisatoren können Metallsatze von Fettsäuren wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quatemiertes Chitosan, Polyvinylpyrrolidon, Vinyl-pyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als Quellmittel für wäßrige Phasen können Montmorillonite, Clay Mineralstoffe, Pemulen sowie alkyl-modifizierte Carbopoltypen (Goodrich) dienen.

Unter UV-Lichtschutzfiltern sind organische Substanzen zu verstehen, die in der Lage sind, ultraviolette Strahlen zu absorbieren und die aufgenommene Energie in Form längerwelliger Strahlung, z.B. Wärme wieder abzugeben. Typische Beispiele sind 4-Aminobenzoesäure sowie ihre Ester und Derivate (z.B. 2-Ethylhexyl-p-dimethylaminobenzoat oder p-Dimethylaminobenzoesäureoctylester), Methoxyzimtsäure und ihre Derivate (z.B. 4-Methoxyzimtsäure-2-ethylhexylester), Benzophenone (z.B. Oxybenzon, 2-Hydroxy-4-methoxybenzophenon), Dibenzoylmethane, Salicylatester, 2-Phenylbenzimidazol-5-sulfonsäure, 1-(4-tert.Butylphenyl)-3-(4'-methoxyphenyl)-propan-1,3-dion, 3-(4'-Methyl)benzylidenbornan-2-on, Methylbenzylidencampher und dergleichen. Weiterhin kommen für diesen Zweck auch feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Zur Verbesserung des Fließverhaltens können ferner **Hydrotrope** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylofbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Als **Konservierungsmittel** eignen sich beispielsweise Phenoxyethanol, Formaldehydlösung, Parabene, Pentandiol oder Sorbinsäure. Als Insekten-Repellentien kommen N,N-Diethyl-m-touluamid, 1,2-Pentandiol oder Insect repellent 3535 in Frage, als **Selbstbräuner** eignet sich Dihydroxyaceton. Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt. Der Gesamtanteil der Hilfs- und Zusatzstoffe kann 1 bis 50, vorzugsweise 5 bis 40 Gew.-% - bezogen auf die Mikrosphären - betragen.

### Fällung von Chitosan/Ölkörpermischungen

Ein weiterer Gegenstand der Erfindung betrifft ein Verfahren zur Herstellung von Chitosanmikrosphären, bei dem man Chitosane und/oder Chitosanderivate mit Ölkörpem vermischt und diese dann in alkalisch eingestellte wäßrige Tensidlösungen einbringt. Hierzu wird eine Lösung des Chitosans bzw. Chitosanderivats in Wasser oder einer Wasser/Glycolsäure-Mischung vorgelegt und unter starker Scherung mit dem Ölkörper sowie gegebenenfalls den weiteren Zusatzstoffen zu einer Dispersion verarbeitet. Falls erforderlich, kann man die Löslichkeit der lipophilen Phase im Chitosan durch Zusatz geeigneter Lösungsvermittler, wie beispielsweise Ethanol, verbessem; die Einsatzmenge der Hydrotrope kann im Bereich von 10 bis 100, vorzugsweise 20 bis 50 Gew.-% bezogen auf die Chitosane liegen. In gleicher Weise kann man auch Emulsionen verkapseln. Bei dem erfindungsgemäßen Verfahren werden die Chitosandispersionen bzw. -emulsionen in die wäßrigen Tensidlösungen eingetropft, wobei Mikrosphären in Form von Perlen mit einem Durchmesser von 1 bis 5 mm entstehen, die die lipophile (Wirkstoff-)Phase im Inneren enthalten.

### Tenside

Bei dem Fällungsmittel kann es sich um wäßrige Lösungen von anionischen, nichtionischen, kationischen und/oder amphoteren bzw. zwitterionischen Tensiden handeln, die einen Feststoffgehalt im Bereich von 1 bis 50, vorzugsweise 5 bis 35 Gew.-% aufweisen. Typische Beispiele für anionische **Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls. partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen und Esterquats, insbesondere quatemierte Fettsäuretrialkanolaminestersalze. Typische Beispiele für **amphotere bzw. zwitterionische Tenside** sind Alkylbetaine, Alkylamidobetaine, Aminopropionate, Aminoglycinate, imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124 oder J.Falbe (ed.), "Katalysatoren, Tenside und Mineratöladditive", Thieme Verlag, Stuttgart, 1978, S. 123-217** verwiesen. Üblicherweise werden Tensidlösungen eingesetzt, die einen pH-Wert im Bereich von 7,5 bis 10, vorzugsweise 8 bis 9 aufweisen. Demzufolge kann man Tenside, die unter alkalischen Bedingungen nicht längere Zeit stabil sind, wie beispielsweise estergruppenhaltige Verbindungen, zwar einsetzen, ihre Verwendung ist jedoch weniger bevorzugt Vorzugsweise werden milde Tenside wie beispielsweise Alkylethersulfate, Alkylpolyglucoside und/oder Betaine eingesetzt.

### Gewerbliche Anwendbarkeit

Die erfindungsgemäßen Chitosanmikrosphären weisen nicht nur ein ästhetisches Erscheinungsbild auf, sondern geben die im Innern gespeicherte lipophile Phase, die zudem auch eine Vielzahl von Wirkstoffen für die Pharmazie enthalten kann, zeitverzögert und sehr gleichmäßig frei. Ein weiterer Gegenstand der Erfindung betrifft daher die Verwendung der Chitosanmikrosphären als Wirkstoffdepots zur Herstellung von kosmetischen und/oder pharmazeutischen Zubereitungen, in denen sie in Mengen von 1 bis 50, vorzugsweise 5 bis 25 Gew.-%- bezogen auf die Mittel - enthalten sein können.

### Beispiele

In einer Rührapparatur wurden unter starker Scherung wäßrige Chitosanlösungen (Feststoffgehalt 1 Gew.-%) und Ölkörper sowie gegebenenfalls Lösungsvermittler zu Dispersionen verarbeitet. Diese wurden anschließend tropfenweise in alkalische eingestellte Tensidlösungen (Feststoffgehalt 15 Gew.-%) eingebracht. Die Mikrosphären wurden unmittelbar in Form von Perlen mit einem Durchmesser von . 1 bis 5 mm gefällt und konnten durch Abdekantieren der überstehenden wäßrigen Phase erhalten werden. Die Einzelheiten der Versuche sind in Tabelle 1 zusammengefaßt.

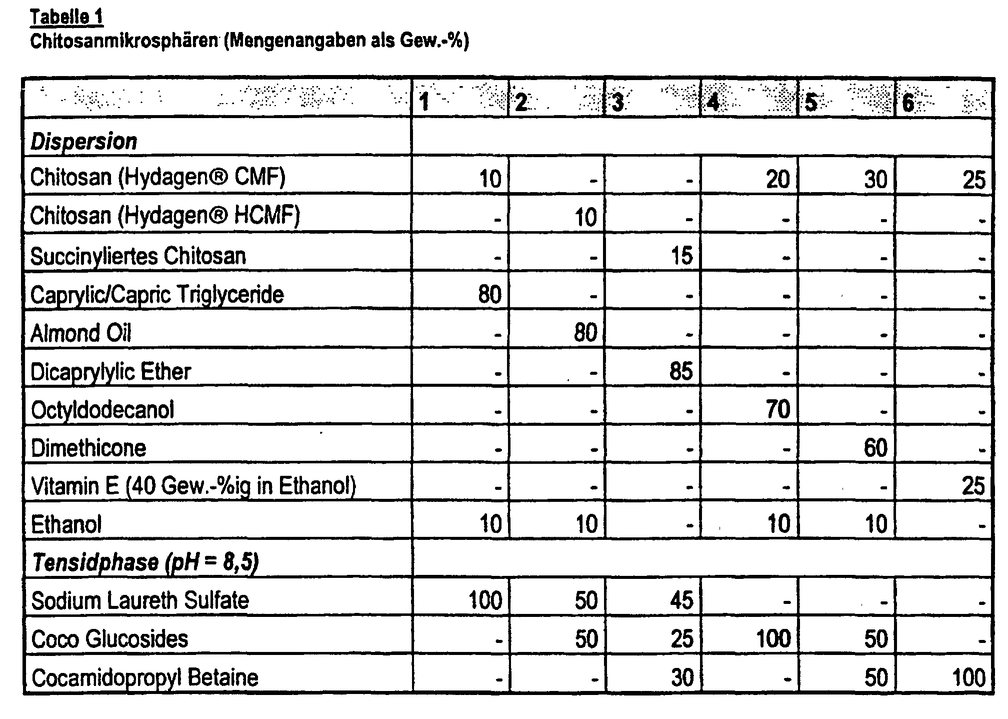

## Patentansprüche

1. Chitosanmikrosphären, dadurch erhältlich, daß man Chitosane und/oder Chitosanderivate mit Ölkörpem vermischt und die Mischungen anschließend in alkalisch eingestellte Tensidlösungen einbringt, so daß die resultierenden Chitosanmikrosphären einen Durchmesser von 1 mm bis 5 mm aufweisen.

2. Chitosanmikrosphären nach Anspruch 1, **dadurch gekennzeichnet, daß** sie Chitosane mit einem durchschnittlichen Molekulargewicht von 800.000 bis 1200.000 Dalton, einer Viskosität nach Brookfield (1 Gew.-%ig in Glycolsäure) unterhalb von 5000 mPas, einem Deacetylierungsgrad im Bereich von 80 bis 88 % und einen Aschegehalt von weniger als 0,3 Gew.-% enthalten.

3. Chitosanmikrosphären nach den Ansprüchen 1 und 2, **dadurch gekennzeichnet, daß** sie Carboxylierungs-, Succinylierungs- oder Alkoxylierungsprodukte von Chitosanen enthalten.

4. Chitosanmikrosphären nach den Ansprüchen 1 bis 3, **dadurch gekennzeichnet, daß** sie Ölkörper enthalten, die ausgewählt sind aus der Gruppe, die gebildet wird von Guerbetalkoholen auf Basis von Fettalkoholen mit 6 bis 18 Kohlenstoffatomen, Estern von linearen C₆-C₂₂-Fettsäuren mit linearen C₆-C₂₂-Fettalkoholen, Estern von verzweigten C₆-C₁₃-Carbonsäuren mit linearen C₆-C₂₂-Fettalkoholen, Estern von linearen C₆-C₂₂-Fettsäuren mit verzweigten Alkoholen, Estern von linearen und/oder verzweigten Fettsäuren mit mehrwertigen Alkoholen und/oder Guerbetalkoholen, Triglyceriden auf Basis C₆-C₁₀-Fettsäuren, flüssigen Mono-/Di-/Triglyceridmischungen auf Basis von C₆-C₁₈-Fettsäuren, Estem von C₆-C₂₂-Fettalkoholen und/oder Guerbetalkoholen mit aromatischen Carbonsäuren, pflanzliche Ölen, verzweigten primären Alkoholen, substituierten Cyclohexanen, linearen C₆-C₂₂-Fettalkoholcarbonaten, Guerbetcarbonaten, Estem der Benzoesäure mit linearen und/oder verzweigten C₆-C₂₂-Alkoholen, Dialkylethem, Siliconölen und/oder aliphatischen bzw. naphthenischen Kohlenwasserstoffen.

5. Chitosanmikrosphären nach den Ansprüchen 1 bis 4, **dadurch gekennzeichnet, daß** sie die Chitosane und die Ölkörper im Gewichtsverhältnis 1 : 10 bis 10:1 enthalten.

6. Chitosanmikrosphären nach den Ansprüchen 1 bis 5, **dadurch gekennzeichnet, daß** sie als weitere Hilfs- und Zusatzstoffe Emulgatoren, Überfettungsmittel, Stabilisatoren, Wachse, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Lichtschutzfilter, Insektenrepellentien, Selbstbräuner, Farb- und Duftstoffe enthalten.

7. Verfahren zur Herstellung von Chitosanmikrosphären, bei dem man Chitosane und/oder Chitosanderivate mit Ölkörpem vermischt und diese dann in alkalisch eingestellte wäßrige Tensidlösungen einbringt, so daß die resultierenden Chitosanmikrosphären einen Durchmesser von 1 mm bis 5 mm aufweisen.

8. Verfahren nach Anspruch 7, **dadurch gekennzeichnet, daß** man Lösungen von anionischen, nichtionischen, kationischen und/oder amphoteren bzw. zwitterionischen Tensiden einsetzt.

9. Verfahren nach den Ansprüchen 7 und 8, **dadurch gekennzeichnet, daß** man Tensidlösungen einsetzt, die einen pH-Wert im Bereich von 7,5 bis 10 aufweisen.

10. Verwendung von Chitosanmikrosphären nach Anspruch 1 als Wirkstoffdepots zur Herstellung von kosmetischen und/oder pharmazeutischen Zubereitungen.

## Claims

1. Chitosan microspheres obtainable by mixing chitosans and/or chitosan derivatives with oils and introducing the resulting mixtures into alkaline surfactant solutions so that the resulting chitosan microspheres have a diameter of 1 mm to 5 mm.

2. Chitosan microspheres as claimed in claim 1, **characterized in that** they contain chitosans with an average molecular weight of 800,000 to 1,200,000 dalton, a Brookfield viscosity (1% by weight in glycolic acid) below 5,000 mPas, a degree of deacetylation of 80 to 88% and an ash content of less than 0.3% by weight.

3. Chitosan microspheres as claimed in claims 1 and 2, **characterized in that** they contain carboxylation, succinylation or alkoxylation products of chitosans.

4. Chitosan microspheres as claimed in claims 1 to 3, **characterized in that** they contain oils selected from the group consisting of Guerbet alcohols based on fatty alcohols containing 6 to 18 carbon atoms, esters of linear C₆₋₂₂ fatty acids with linear C₆₋₂₂ fatty alcohols, esters of branched C₆₋₁₃ carboxylic acids with linear C₆₋₂₂ fatty alcohols, esters of linear C₆₋₂₂ fatty acids with branched alcohols, esters of linear and/or branched fatty acids with polyhydric alcohols and/or Guerbet alcohols, triglycerides based on C₆₋₁₀ fatty acids, liquid mono-/di-/triglyceride mixtures based on C₆₋₁₈ fatty acids, esters of C₆₋₂₂ fatty alcohols and/or Guerbet alcohols with aromatic carboxylic acids, vegetable oils, branched primary alcohols, substituted cyclohexanes, linear C₆₋₂₂ fatty alcohol carbonates, Guerbet carbonates, esters of benzoic acid with linear and/or branched C₆₋₂₂ alcohols, dialkyl ethers, silicone oils and/or aliphatic or naphthenic hydrocarbons.

5. Chitosan microspheres as claimed in claims 1 to 4, **characterized in that** they contain the chitosans and the oils in a ratio by weight of 1:10 to 10:1.

6. Chitosan microspheres as claimed in claims 1 to 5, **characterized in that** they contain as further auxiliaries and additives emulsifiers, superfatting agents, stabilizers, waxes, consistency providers, thickeners, cationic polymers, silicone compounds, biogenic agents, antidandruff agents, film formers, preservatives, hydrotropes, solubilizers, UV filters, insect repellents, self-tanning agents, dyes and perfumes.

7. A process for the production of chitosan microspheres in which chitosans and/or chitosan derivatives are mixed with oils and the resulting mixture is introduced into alkaline aqueous surfactant solutions so that the resulting chitosan microspheres have a diameter of 1 mm to 5 mm.

8. A process as claimed in claim 7, **characterized in that** solutions of anionic, nonionic, cationic and/or amphoteric or zwitterionic surfactants are used.

9. A process as claimed in claims 7 and 8, **characterized in that** surfactant solutions with a pH value of 7.5 to 10 are used.

10. The use of the chitosan microspheres claimed in claim 1 as active-substance reservoirs for the production of cosmetic and/or pharmaceutical preparations.

## Revendications

1. Microsphères de chitosanes que l'on peut obtenir en mélangeant des chitosanes et/ou des dérivés de chitosanes avec des corps huileux et en incorporant ensuite ces mélanges dans des solutions tensioactives rendues alcalines de sorte que les microsphères de chitosanes qui en résultent aient un diamètre de 1 à 5 mm.

2. Microsphères de chitosanes selon la revendication 1,
**caractérisées en ce qu'**
elles renferment des chitosanes d'un poids moléculaire moyen de 800.000 à 1.200.000 Dalton, d'une viscosité selon Brookfield (à 1% en poids dans de l'acide glycolique) inférieure à 5000 mPas, d'un taux de désacétylation de l'ordre de 80 à 88% en poids et d'une teneur en cendres inférieure à 0,3% en poids.

3. Microsphères de chitosanes selon les revendications 1 et 2,
**caractérisées en ce qu'**
elles renferment des produits de la carboxylation, de succinylation ou d'alcoxylation de chitosanes.

4. Microsphères de chitosanes selon les revendications 1 à 3,
**caractérisées en ce qu'**
elles renferment des corps huileux choisis dans le groupe constitué d'alcools de Guerbet à base d'alcools gras portant 6 à 18 atomes de carbone, d'esters d'acides gras linéaires en C₆ à C₂₂ et d'alcools gras linéaires en C₆ à C₂₂, d'esters d'acides carboxyliques ramifiés en C₆ à C₁₃ et d'alcools gras linéaires en C₆ à C₂₂, d'esters d'acides gras linéaires en C₆ à C₂₂ et d'alcools ramifiés, d'esters d'acides gras linéaires et/ou ramifiés et d'alcools polyvalents et/ou d'alcools de Guerbet, de triglycérides à base d'acides gras en C₆ à C₁₀, de mélanges de mono-/, di-/triglycérides liquides à base d'acides gras en C₆ à C₁₈, d'esters d'alcools gras en C₆ à C₂₂ et/ou d'alcools de Guerbet et d'acides carboxyliques aromatiques, d'huiles végétales, d'alcools primaires ramifiés, de cyclohexanes substitués, de carbonates d'alcools gras linéaires en C₆ à C₂₂, de carbonates de Guerbet, d'esters de l'acide benzoïque et d'alcools linéaires et/ou ramifiés en C₆ à C₂₂, d'éthers dialkyliques, d'huiles silicones et/ou d'hydrocarbures aliphatiques ou naphténiques.

5. Microsphères de chitosanes selon les revendications 1 à 4,
**caractérisées en ce qu'**
elles renferment les chitosanes et les corps huileux dans un rapport pondéral de 1/ 10 à 10/1.

6. Microsphères de chitosanes selon les revendications 1 à 5,
**caractérisées en ce qu'**
elles renferment en tant qu'autres excipients et additifs, des agents émulsifiants, des agents surgraissants, des stabilisants, des cires, des agents de consistance, des épaississants, des polymères cationiques, des composés de silicones, des agents actifs biogènes, des agents antipelliculaires, des agents filmogènes, des agents de conservation, des hydrotropes, des solubilisants, des filtres photoprotecteurs anti-UV, des répulsifs pour insectes, des auto-bronzants, des colorants et des substances odorantes.

7. Procédé de fabrication de microsphères de chitosanes selon lequel on mélange des chitosanes et/ou des dérivés de chitosanes avec des corps huileux et on les introduit ensuite dans des solutions tensioactives aqueuses rendues alcalines de manière à ce que les microsphères de chitosanes qui en résultent aient un diamètre de 1 à 5 mm.

8. Procédé selon la revendication 7,
**caractérisé en ce qu'**
on met en oeuvre des solutions de tensioactifs anioniques, non ioniques, cationiques et/ou amphotères ou zwitterioniques.

9. Procédé selon les revendications 7 à 8,
**caractérisé en ce qu'**
on met en oeuvre des solutions tensioactives qui ont un pH de l'ordre de 7,5 à 10.

10. Utilisation de microsphères de chitosanes selon la revendication 1 pour rassembler des agents actifs dans la fabrication de préparations cosmétiques et/ou pharmaceutiques.
